# EUROPEAN PATENT APPLICATION

(11) **EP 4 306 106 A1**
(43) Date of publication of application: **17.01.2024**
(21) Application number: 23185697.2
(22) Date of filing: 14.07.2023
(51) Int. Cl.: A61K 31/12, A61K 31/44, A61P 25/00, A61P 35/00

(54) **NITRO-CONTAINING COMPOUNDS, COMPOSITIONS AND USES THEREOF**

(30) Priority: 14.07.2022 PT 2022118107; 11.08.2022 PT 2022118158; 12.08.2022 EP 22190310
(71) Applicant: Universidade de Aveiro, 3810-193 Aveiro (PT)
(72) Inventor: SOARES DA SILVA, ARTUR MANUEL, 3810-193 AVEIRO (PT); NEVES FRANCISCO, TELMO, 3810-193 AVEIRO (PT); CARDOSO DE SOUSA, JOANA LIA, 3810-193 AVEIRO (PT); TEIXEIRA ALBUQUERQUE, HÉLIO MIGUEL, 3810-193 AVEIRO (PT)
(74) Representative: Patentree

(57) **Abstract**

The present disclosure relates to nitro-containing compounds capable of inhibiting histone deacetylases (HDACs), with selectivity for HDAC8, and their use in medicine, namely as therapeutic agents in oncology or neurodegenerative diseases. Furthermore, the present disclosure relates to compositions and to methods to obtain such compounds.

## Description

### TECHNICAL FIELD

The present disclosure relates to a particular type of nitro-containing compounds capable of inhibiting histone deacetylases (HDACs), with selectivity for HDAC8, and their use in medicine, namely as therapeutic agents in oncology. Furthermore, the present disclosure relates to compositions and methods to obtain such compounds.

### BACKGROUND

Epigenetics is classically defined as the reversible changes in gene expression that do not result from changing DNA sequences, which has been found to play an important role in the origin, development, and metastasis of cancer. Epigenetic writer, eraser, and reader enzymes, including histone deacetylases (HDACs), DNA methyltransferases, and histone methyltransferases, have received increasing attention, being often studied as targets for drug discovery in cancer therapy.¹

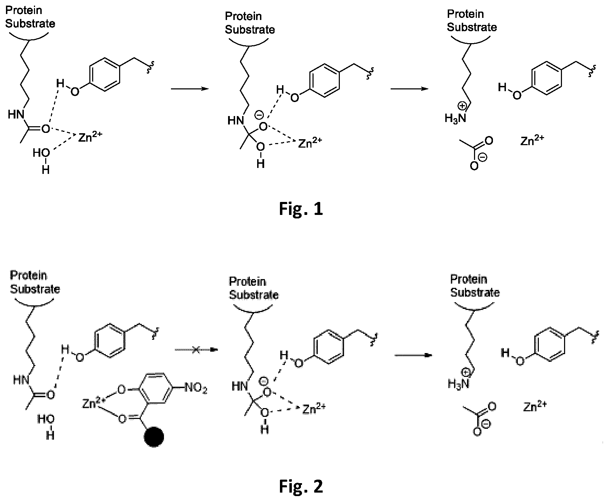

Histone deacetylases (HDACs) are a class of enzymes with deacetylase activity with a broad range of genomic and nongenomic substrates. In humans, there are 18 HDACs that fall into two families based on their catalytic mechanism. Eleven of the HDACs are zinc-dependent metalloenzymes named HDAC1-11 capable of hydrolysing the amide bond using water as a nucleophile. The remaining seven sirtuins 1-7 employ NAD⁺ as a cofactor and transfer the acyl group to the C-2 position of the ribose sugar. Despite both enzyme families perform the same chemical reaction of acyllysine cleavage, the term HDAC usually refers to the zinc dependent enzymes. Extensive mechanistic studies, exemplified for HDAC8 **(****Figure 1****),** have provided a detailed understanding of the catalytic mechanism.¹

The acetyllysine substrate sits in a narrow channel lined with hydrophobic residues, with a tyrosine residue flipping in conformation to enabling hydrogen bonding with the carbonyl oxygen. A Zn²⁺ cation, coordinated to aspartate and histidine residues, lies at the end of the substrate channel, bound to a water molecule activated by a charge relay mechanism with two adjacent histidine residues. Then, water nucleophilic attack to carbonyl group by the generates a tetrahedral oxyanion intermediate.

HDAC inhibitors have been described as therapeutic agents in oncology, neurodegeneration, autoimmune disease, chemotherapy-induced peripheral neuropathy, and cardiac indications, but they often suffer from nonspecific binding leading to significant adverse effects such as fatigue, nausea, diarrhoea, and thrombocytopenia. Structurally, one can find common pharmacophores such as aromatic rings, zinc binding groups and caps, as well as conjugated and nonconjugated linkers. However, there is no example of HDAC inhibitors having the nitro group, specifically the nitrobenzene pharmacophore.²⁻⁴

Indeed, selective inhibition of HDACs has shown promising results in various disease contexts. The potential therapeutic effects of HDAC inhibitors are due to modulation of the acetylation levels of histones and other proteins, which can impact gene expression and cellular processes. Diseases that have already shown to be improved or prevented through selective HDAC inhibition include cancer, and neurological disorders. For cancer, HDAC inhibitors have been extensively studied and used as potential anticancer agents. By modulating gene expression and promoting the acetylation of histones, these inhibitors can induce cell cycle arrest, promote differentiation, and trigger apoptosis in cancer cells. Some HDAC inhibitors, such as vorinostat and romidepsin, have been approved for the treatment of certain types of lymphomas and cutaneous T-cell lymphoma. Regarding neurological disorders, selective HDAC inhibition has shown promise in several neurological disorders, including neurodegenerative diseases and psychiatric conditions. HDAC inhibitors have been investigated for their potential to enhance memory and cognition in Alzheimer's disease and other forms of dementia. Additionally, they have demonstrated potential antidepressant and antianxiety effects in preclinical studies.

The commonly used HDAC inhibitors have a hydroxamic acid (HA) functionality to chelate the Zn²⁺ ion in the catalytic pocket. This is the gold-standard mode of action of HA-based inhibitors. Despite their powerful HDAC inhibitor activity, concerns have been raised about the clinical use of HA-based HDAC inhibitors. They have been presenting severe cardiac side effects, as well as poor pharmacokinetics (PK) as a result of rapid clearance. Therefore, there is a high need to identify powerful HA surrogates with alternative zinc-binding groups (ZBG) as a means of providing safer HDACi that could also be included in (long-term) non-oncological applications (Ver ref 6). Herein, an alternative ZBG is disclosed for the first time, based on the 2-hydroxy-5-nitrophenylketone moiety, which complex Zn²⁺ blocking the whole subsequent deacetylation process (Fig. 2).

These facts are disclosed in order to illustrate the technical problem addressed by the present disclosure.

### GENERAL DESCRIPTION

The present disclosure relates to nitro group-containing compounds, which present high *in vitro* inhibitory effects towards HDACs, designed differently from the classical pharmacophoric architecture of previous HDAC inhibitors, selectively targeting HDAC8 and consequently, avoiding adverse effects from non-specificity. The present disclosure relates to a family of compounds designed to target HDACs with special emphasis in HDAC8. The present disclosure also relates to the synthesis of the two sets of nitro-containing compounds through new simple and scalable methods, and also, to compositions comprising such compounds. The nitro-containing compounds now disclosed may be used to produce more effective drugs for cancer therapy.

An aspect of the present disclosure relates to a compound of formula I or formula II, or a pharmaceutically acceptable salt, or ester or solvate thereof 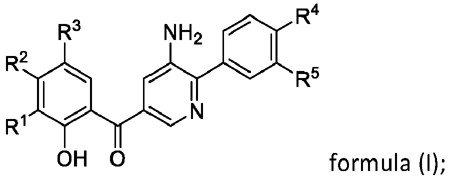 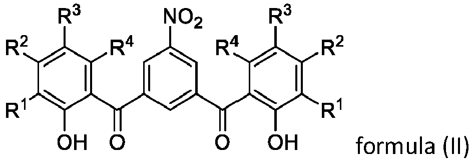 wherein
R¹, R², R³, R⁴ and R⁵ are independently selected from each other;
R¹, R², R³, R⁴ and R⁵ are selected from H, F, Cl, Br, I, CN, NH₂, NO₂, OCH₃, CH₃ or CH₂CH₃; provided that at least R¹, or R², or R³, or R⁴ or R⁵ is NO₂; preferably at least two of R¹, or R², or R³, or R⁴ are NO₂; more preferably at least three of R¹, or R², or R³, or R⁴ are NO₂ for use in medicine. In an embodiment for better results, R¹ is H.

In an embodiment for better results, R¹ is H; R², R³, R⁴ and R⁵ are independently selected from each other; R², R³, R⁴ and R⁵ are selected from H, F, Cl, Br, I, CN, NH₂, NO₂, OCH₃, CH₃ or CH₂CH₃; provided that at least R², or R³, or R⁴ or R⁵ is NO₂.

In an embodiment, the compounds of formula II can be obtained as described in Synlett 2022, 33, 1505-1510.

Another aspect of the present disclosure relates to a compound of formula I, or a pharmaceutically acceptable salt, or ester or solvate thereof 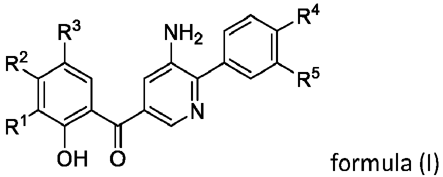 wherein
R¹, R², R³, R⁴ and R⁵ are independently selected from each other;
R¹, R², R³, R⁴ and R⁵ are selected from H, F, Cl, Br, I, CN, NH₂, NO₂, OCH₃, CH₃ or CH₂CH₃; provided that at least R¹, or R², or R³, or R⁴ or R⁵ is NO₂; preferably at least two of R¹, or R², or R³, or R⁴ are NO₂; more preferably at least three of R¹, or R², or R³, or R⁴ are NO₂. In an embodiment for better results, R¹ is H.

In an embodiment for better results, R¹ is H; R², R³, R⁴ and R⁵ are independently selected from each other; R², R³, R⁴ and R⁵ are selected from H, F, Cl, Br, I, CN, NH₂, NO₂, OCH₃, CH₃ or CH₂CH₃; provided that at least R², or R³, or R⁴ or R⁵ is NO₂.

In an embodiment, R³ is NO₂; and R¹, R², R⁴ and R⁵ are selected from H, F, Cl, Br, I, CN, NH₂, OCH₃, CH₃ or CH₂CH₃; preferably R¹ is H and R², R⁴ and R⁵ are selected from H, F, Cl, Br, I, CN, NH₂, OCH₃, CH₃ or CH₂CH₃.

In an embodiment for better results, R¹ is H; R³ is NO₂; and R², R⁴ and R⁵ are selected from H, F, Cl, Br, I, CN, NH₂, OCH₃, CH₃ or CH₂CH₃.

In an embodiment, R⁴ is NO₂ and R¹, R², R³ and R⁵ are selected from H, F, Cl, Br, I, CN, NH₂, OCH₃, CH₃ or CH₂CH₃.

In an embodiment for better results, R¹ is H; R⁴ is NO₂; and R², R³ and R⁵ are selected from H, F, Cl, Br, I, CN, NH₂, OCH₃, CH₃ or CH₂CH₃.

In an embodiment, R³ and R⁴ are NO₂.

In an embodiment for better results, R¹ is H and R³ and R⁴ are NO₂.

In another embodiment, the compound is 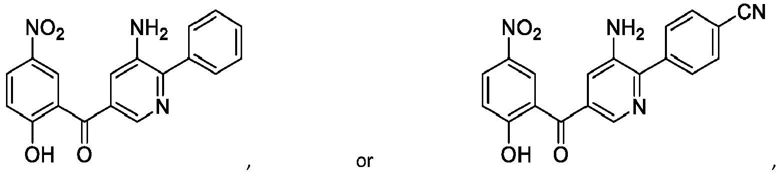 or 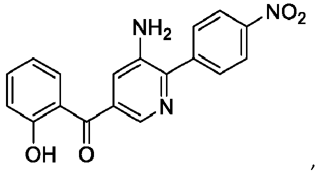 or

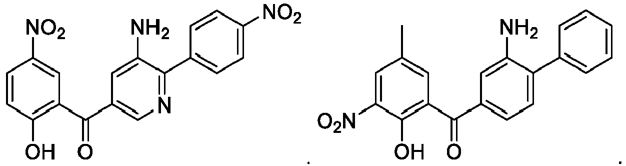

In an embodiment, R³ is NO₂ and R², R⁴ are selected from H, F, Cl, Br, I, CN, NH₂, OCH₃, CH₃ or CH₂CH₃.

In an embodiment for better results, R¹ is H; R³ is NO₂; and R², R⁴ are selected from H, F, Cl, Br, I, CN, NH₂, OCH₃, CH₃ or CH₂CH₃.

In an embodiment, R³ is NO₂.

In an embodiment for better results, R¹ is H; and R³ is NO₂.

In an embodiment, R³ is NO₂ and R⁴ is CH₃.

In an embodiment for better results, R¹ is H; R³ is NO₂ and R⁴ is OCH₃.

In an embodiment, the compound is

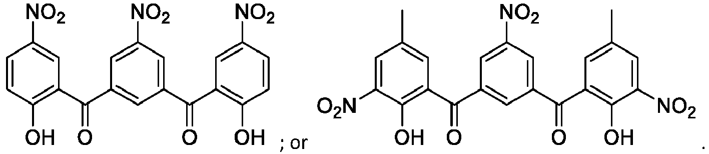

The present disclosure also relates to any compound, or related ones, now disclosed for use in medicine or veterinary.

In an embodiment, the disclosed compounds, or related ones, may be used for any condition susceptible of being improved or prevented by selective inhibition of histone deacetylases.

In an embodiment, the disclosed compounds, or related ones, may be used for any condition susceptible of being improved or prevented by selective inhibition of histone deacetylase 8; preferably selective histone deacetylase 8 inhibitor.

In an embodiment, the disclosed compounds, or related ones, may be used for the prevention or treatment of a neoplasia, preferably for use in the prevention or treatment of cancer.

In an embodiment, the disclosed compounds, or related ones, may be used for the prevention or treatment of a degenerative disease, preferably a neurodegenerative disease.

Another aspect of the present disclosure relates to a pharmaceutical composition comprising the disclosed compounds or related ones, and a pharmaceutically acceptable carrier, wherein the compound is in a therapeutically effective amount.

Another aspect of the present disclosure relates to a method of obtaining the disclosed compound of formula I, or related ones, comprising the following steps:
mixing a 3-formylchromone, a Kröhnke salt and ammonium acetate in an organic solvent;
heating the resulting mixture, preferably under microwave irradiation; wherein the temperature of heating ranges from 90-200°C and in a period ranging from 5-15 min; preferably 180 °C during 15 min, to obtain the crude solution;
evaporating the organic solvent from the crude solution to obtain dried crude;
purifying the obtained dried crude to obtain pure compound; preferably purifying by silica gel flash chromatography.

In another embodiment, the disclosed compound of formula I, or related ones, can be obtained by a method comprising the following steps:
mixing a 3-formylchromone, a Kröhnke salt and ammonium hydroxide in an organic solvent;
heating the resulting mixture, preferably under microwave irradiation; wherein the temperature of heating ranges from 90-200°C and in a period ranging from 5-15 min; preferably 110 °C during 10 min, to obtain the crude solution;
evaporating the organic solvent from the crude solution to obtain dried crude;
purifying the obtained dried crude to obtain pure compound; preferably purifying by silica gel flash chromatography.

In an embodiment, the 3-formylchromone is selected from a list consisting of: 6-nitro-3-formylchromone, 5-methoxy-3-formylchromone, 6-methyl-3-formylchromone, 6-chloro-7-methyl-3-formylchromone, 6-chloro-3-formylchromone, 6-bromo-3-formylchromone, 8-nitro-6-methyl-3-formylchromone, 8-nitro-6-bromo-3-formylchromone or mixtures thereof. Preferably, the 3-formylchromone is selected from a list consisting of: 3-formylchromone, 6-nitro-3-formylchromone, 8-nitro-6-methyl-3-formylchromone, 8-nitro-6-bromo-3-formylchromone.

In an embodiment, the Kröhnke salt is selected from a list consisting of R-containing salts, wherein R is H, Cl, CH₃, OCH₃, CN, NO₂, CF₃; dioxane; naphthalene; pyrene; thiophene; coumarin; or mixtures thereof. Preferably, the Kröhnke salt is selected from a list consisting of R-containing salts, wherein R is H, CN, NO₂, CF₃, or mixtures thereof.

In an embodiment, the organic solvent is selected from a list consisting of: absolute alcohol, methanol, hexafluoroisopropanol (HFIP), acetic acid, tetrahydrofuran (THF), acetonitrile, toluene, phenol, dimethylformamide (DMF); more preferably, absolute ethanol.

In an embodiment, the temperature of heating ranges from 90-180°C, preferably 90-150°C, more preferably 90-130°.

In an embodiment, the pressure of the reaction ranges from 1.38 MPa (200psi) to 1.72 MPa (250psi); preferably 1.52 MPa(220psi).

The compounds herein disclosed present the following advantages in comparison with the already known HDAC inhibitors:
- First-in-class HDAC inhibitors from phenotypic analysis;
- Selectivity for HDAC8;
- The presence in their overall structure of the pharmacophore 1-(2-hydroxy-5-nitrophenyl)ketone (alternative zinc-binding group);
- The 1-(2-hydroxy-5-nitrophenyl)ketone moiety complex Zinc, inhibiting HDACs;
- One-step simple and straightforward synthesis with yield up to 85%, preferably up to 90% (milligram-scale);
- Scalable synthetic method (multigram-scale) with consistent yield (85%);
- Simple purification and high purity from the reaction crude.

The present disclosure also relates to the synthesis of the above-mentioned compounds and their use as HDAC inhibitors. The production of such compounds is easy, and presents good yields, is economical and the compounds are very stable in its powder formulation.

The present disclosure also relates to nitro-containing compounds having the general formula (III), their synthesis and use as HDAC inhibitors, particularly as HDAC8 selective inhibitors: 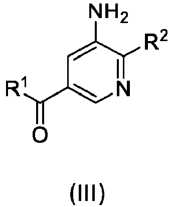 wherein
R¹ is selected from the following list: NO₂-Phenol, Me-Phenol, Halogen-Phenol, OMe-Phenol, naphthyl, alkyl groups, alkenyl, heteroaromatic;
R² is selected from the following list: Ph, NO₂-Ph, CN-Ph, Me-Ph, OMe-Ph, naphthyl, alkyl groups, alkenyl, heteroaromatic;
for use as HDAC inhibitors for epigenetic cancer therapy.

The present disclosure also relates to another set of nitro-containing compounds of general formula (IV),[1] their synthesis and use as HDAC inhibitors, particularly as HDAC8 selective inhibitors. 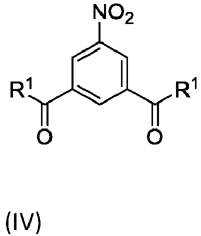 wherein
R¹ is selected from the following list: NO₂-Phenol, Me-Phenol, Halogen-Phenol, OMe-Phenol, naphthyl, alkyl groups, alkenyl, heteroaromatic;
for use as HDAC inhibitors for epigenetic cancer therapy.

The present disclosure also relates to a set of compounds of general formula (V) and (VI), wherein R¹ and R² can be H, Me, OMe, halogen, NO₂, CN. These have been synthesized in large quantities, in particular, on multiple grams scale, starting from a common building block. The building blocks were obtained commercially or synthetically.

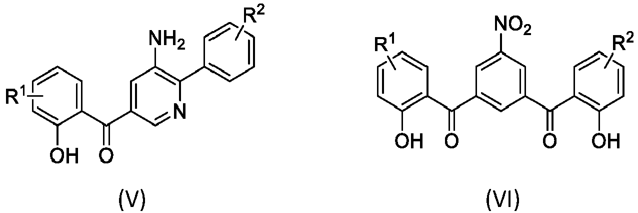

### BRIEF DESCRIPTION OF THE DRAWINGS

The following figures provide preferred embodiments for illustrating the disclosure and should not be seen as limiting the scope of invention.
**Figure 1****:** Simplified illustration of the catalytic mechanism of deacetylation by HDACs.
**Figure 2****:** Simplified illustration of the mechanism of action of the disclosed compounds (alternative zinc-binding group).
**Figure 3**: Embodiment of results of cell viability after 24, 48 and 72h of treatment of Jurkat cells with nitrobenzene and 3-aminopyridine derivatives at different concentrations (0, 0.1 µM, 1 µM), 10 µM, 50 µM and 100 µM)

### DETAILED DESCRIPTION

The present disclosure relates to nitro-containing compounds capable of inhibiting histone deacetylases (HDACs), with selectivity for HDAC8, and their use in medicine, namely as therapeutic agents in oncology or neurodegenerative diseases. Furthermore, the present disclosure relates to compositions and to methods to obtain such compounds.

In an embodiment, the present disclosure relates to libraries of nitro-containing compounds bearing the amino-pyridine and nitrobenzene moieties, with 2-hydroxy-nitrophenylketones as alternative zinc-binding group, as HDAC inhibitors for the use in epigenetic cancer therapy.

**Table 1** provides examples of nitro-containing compounds herein disclosed:

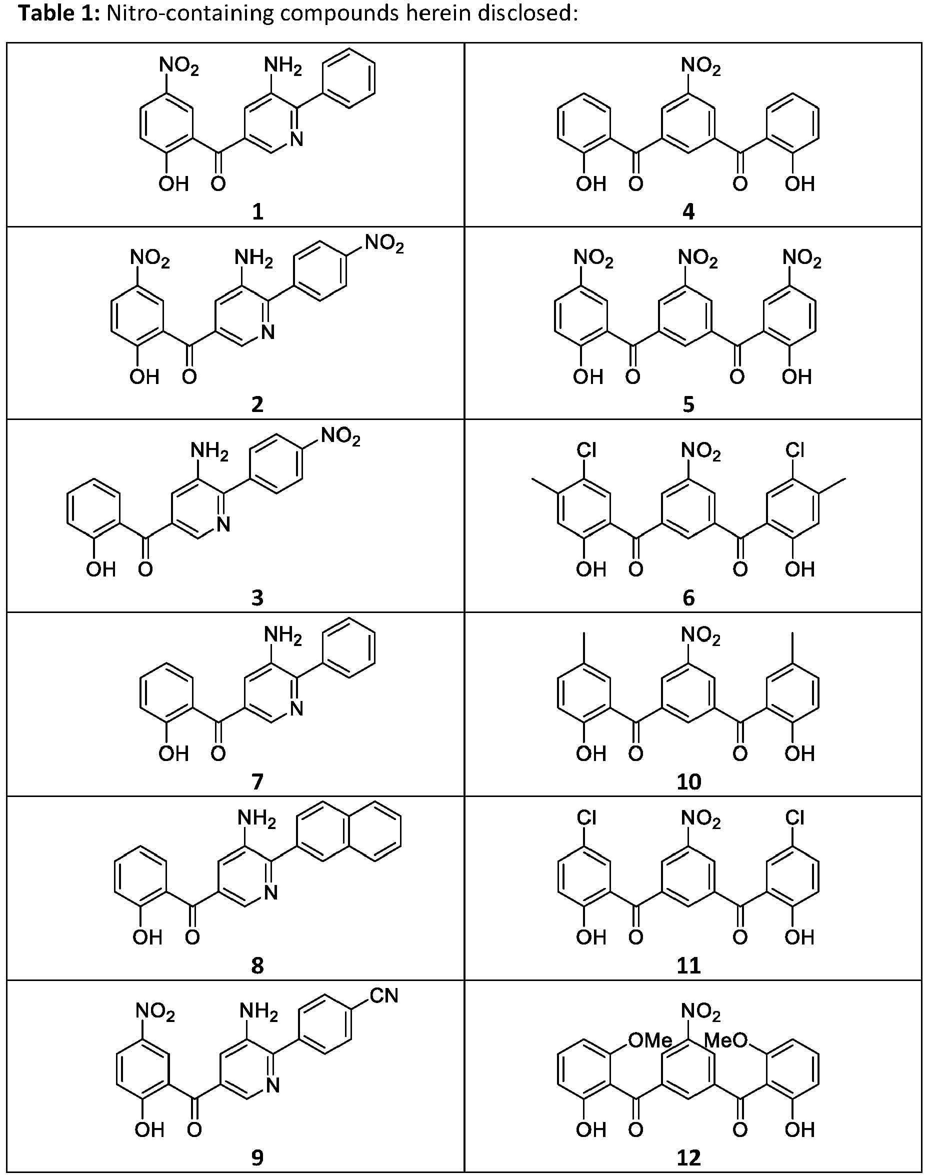

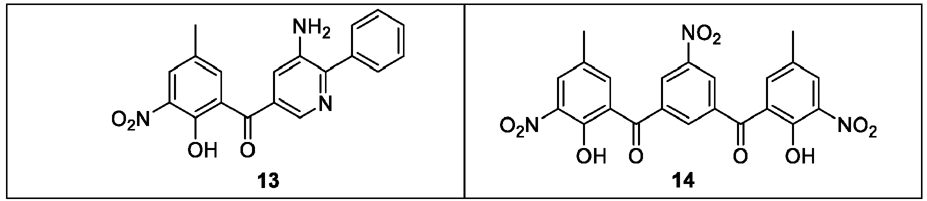

In an embodiment, the synthesis of 3-aminopyridines **1-3** and **7-9** and 13 comprises the following steps:
mixing 1 equivalent of the selected 3-formylchromone, 1.5 equivalents of the selected Kröhnke salt and 15 equivalents of ammonium acetate in absolute ethanol; heating the resulting mixture at 180 °C for 15 minutes under microwave irradiation; evaporating the ethanol; purifying the obtained crude compounds by silica gel flash column chromatography with an eluent, in particular, hexane/EtOAc (5:1).

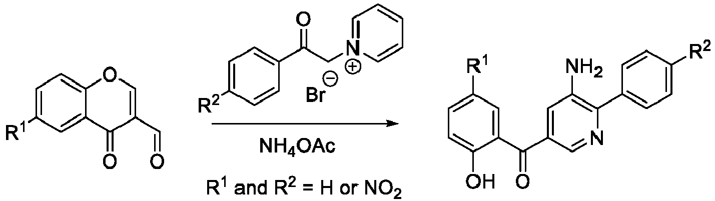

In another embodiment, the synthesis of 3-aminopyridines 1-3 and 7-9 comprises the following steps: mixing 1 equivalent of the selected 3-formylchromone, 1.5 equivalents of the selected Kröhnke salt and 10 equivalents of ammonium hydroxide in absolute ethanol; heating the resulting mixture at 110 °C for 10 minutes under microwave.

The characterization of the compounds 1-3 and 7-9 is now disclosed.

### (5-Amino-6-phenylpyridin-3-yl)(2-hydroxy-5-nitrophenyl)methanone (1)

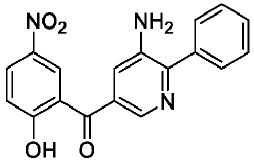

In an embodiment, the characterization of the (5-amino-6-phenylpyridin-3-yl)(2-hydroxy-5-nitrophenyl)methanone compound **(1)** is as follows: a yellow solid product was obtained, with 51% yield, a melting point (m.p.) of 167-169 °C. Further ¹H NMR (300 MHz, CDCl₃): δ 12.57 (s, 1H, 2'-O*H*), 8.71 and 7.37 (2 d, *J* = 2.3 Hz, 1H, H-4,6), 8.45-8.41 (m, 2H, H-4',6'), 7.79-7.76 and 7.58-7.48 (2 m, 5H, H-2",3",4",5",6"), 7.21 (d, *J* = 9.2 Hz, 1H, H-3'), 4.17 (s, 2H, N*H*₂) ppm. ¹³C NMR (75 MHz, CDCl₃): δ 198.7 (C=O), 167.8 (C-2'), 157.7 (C-5'), 149.0 (C-2), 140.3 (C-3), 139.5 (C-6'), 137.3 (C-1"), 131.6 (C-5), 131.3 (C-4'), 129.3 and 121.9 (C-4,6), 129.2 (C-4"), 129.1 and 128.4 (C-2",3",5",6"), 119.7 (C-3'), 118.1 (C-1') ppm.

### [5-Amino-6-(4-nitrophenyl)pyridin-3-yl](2-hydroxy-5-nitrophenyl)methanone (2)

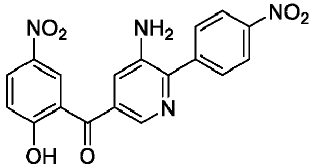

In an embodiment, the characterization of the [5-amino-6-(4-nitrophenyl)pyridin-3-yl](2-hydroxy-5-nitrophenyl)methanone compound (2) is as follows: an orange solid product, with a yield of 17%, a m.p. of 219-220 °C. Further ¹H NMR (300 MHz, DMSO-*d*₆): δ 8.36-8.30 (m, 1H, H-4'), 8.33 (d, J = 8.9 Hz, 2H, H-3",5"), 8.28 (d, *J* = 2.9 Hz, 1H, H-6'), 8.25 (d, *J* = 1.9 Hz, 1H, H-6), 8.03 (d, *J* = 8.9 Hz, 2H, H-2",6"), 7.55 (d, *J* = 1.9 Hz, 1H, H-4), 7.17 (d, *J* = 9.0 Hz, 1H, H-3'), 5.71 (s, 2H, N*H*₂) ppm. ¹³C NMR (75 MHz, DMSO-*d*₆): δ 193.9, 162.1, 147.3, 145.2, 143.9, 143.0, 139.9, 138.8, 132.7, 130.1, 128.7, 126.6, 126.4, 124.1, 123.7, 117.7 ppm.

### [5-Amino-6-(4-nitrophenyl)pyridin-3-yl](2-hydroxyphenyl)methanone (3)

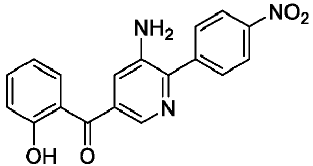

In an embodiment, the characterization of the [5-amino-6-(4-nitrophenyl)pyridin-3-yl](2-hydroxyphenyl)methanone compound **(3)** is as follows: a yellow solid product, with a yield of 55% and a m.p. of 181-182 °C. Further ¹H NMR (300 MHz, DMSO-*d*₆): δ 8.33 (d, *J* = 8.3 Hz, 2H, H-3",5"), 8.20 (s, 1H), 8.03 (d, *J* = 8.3 Hz, 2H, H-2",6"), 7.61 - 7.29 (m, 3H), 7.15-6.70 (m, 2H), 5.69 (s, 2H, N*H*₂) ppm. ¹³C NMR (75 MHz, DMSO-*d₆*): δ 196.9, 158.9, 147.2, 145.4, 143.1, 142.8, 138.6, 134.2, 134.0, 131.0, 130.1, 124.8, 124.1, 123.7, 119.0, 117.7 ppm.

### (5-amino-6-phenylpyridin-3-yl)(2-hydroxyphenyl)methanone (7)

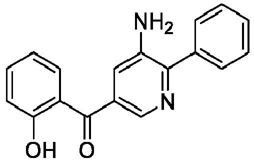

In an embodiment, the characterization of (5-amino-6-phenylpyridin-3-yl)(2-hydroxyphenyl)methanone (7) is as follows: a yellow solid product, with a yield of 72%. Further ¹H NMR (300 MHz, CDCl₃): 11.94 (s, O*H,* 1H), 8.38 (d, *J* = 1.9 Hz, H-1, 1H), 7.78 - 7.67 (m, H-12, 2' and 6', 3H), 7.59 - 7.41 (m, H-10, 3', 4' and 5', 4H), 7.35 (d, *J* = 1.9 Hz, H-3, 1H), 7.09 (dd, *J* = 8.4, 1.2 Hz, H-9, 1H), 6.92 (ddd, *J* = 8.2, 7.2, 1.2 Hz, H-11, 1H), 4.08 (s, N*H*2, 2H) ppm. ¹³C NMR (75 MHz, CDCl₃): δ 199.6 (C-6), 163.2 (C-8), 147.9 (C-5), 140.0 (C-4), 139.8 (C-1), 137.6 (C-1'), 136.8 (C-4'), 133.3 (C-12), 133.0 (C-2), 129.1 (C-10, 3' and 5', 3C), 128.4 (C-2' and 6'), 122.4 (C-3), 119.2 (C-7), 119.0 (C-11), 118.5 (C-9) ppm.

### [5-amino-6-(naphthalen-2-yl)pyridin-3-yl](2-hydroxyphenyl)methanone (8)

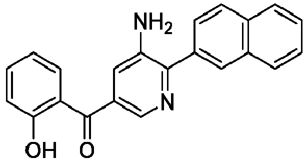

In an embodiment, the characterization of [5-amino-6-(naphthalen-2-yl)pyridin-3-yl](2-hydroxyphenyl)methanone **(8)** is as follows: a yellow solid product, with a yield of 46%. Further ¹H NMR (300 MHz, CDCl₃): δ 11.94 (s, 1H, 2"-O*H*), 8.45 (d, *J* = 1.8 Hz, 1H, H-4), 8.26 (d, *J* = 2.0 Hz, 1H, H-2'), 8.01 (d, *J* = 8.5 Hz, 1H, H-5'), 7.97-7.89 (m, 2H, H-7',10'), 7.86 (dd, *J* = 8.5, 2.0 Hz, 1H, H-6'), 7.74 (dd, *J* = 8.2, 1.7 Hz, 1H, H-6"), 7.60-7.52 (m, 3H, H-4" + H-8',9'), 7.41 (d, *J* = 1.8 Hz, 1H, H-6), 7.11 (dd, *J* = 8.4, 1.2 Hz, 1H, H-3"), 6.94 (ddd, *J* = 8.2, 7.2, 1.2 Hz, 1H, H-5"), 4.17 (s, 2H, 3-N*H*₂) ppm. ¹³C NMR (75 MHz, CDCl₃): δ 199.4 (C-6), 163.2 (C-2"), 147.7 (C-2), 140.3 (C-5), 139.9 (C-4), 136.9 (C-4"), 133.4 (C-3' or C-4' or C-6"), 133.3 (C-3' or C-4' or C-6"), 133.2 (C-3' or C-4' or C-6"), 133.0 (C-3), 129.0 (C-5'), 128.4 (C-7',10'), 127.8 (C-2'), 126.9 (C-8',9'), 126.6 (C-1'), 125.9 (C-6'), 122.6 (C-6), 119.2 (C-1"), 119.0 (C-5"), 118.6 (C-3") ppm.

### 4-[3-amino-5-(2-hydroxy-5-nitrobenzoyl)pyridin-2-yl]benzonitrile (9)

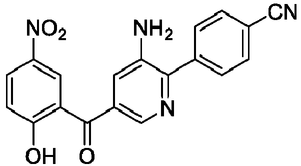

In an embodiment, the characterization of the 4-[3-amino-5-(2-hydroxy-5-nitrobenzoyl)pyridin-2-yl]benzonitrile (9) is as follows: a yellow solid product, with a yield of 65%. Further ¹H NMR (300 MHz, CDCl₃): 12.49 (s, 1H, 2"-OH), 8.67 (d, J = 2.7 Hz, 1H, H-6"), 8.45 (dd, *J* = 9.0, 2.7 Hz, 1H, H-4"), 8.42 (d, *J* = 2.1 Hz, 1H, H-4), 7.99 - 7.90 (m, 2H, H-2',6' or H-3',5'), 7.89 - 7.80 (m, 2H, H-2',6' or H-3',5'), 7.41 (d, *J* = 2.1 Hz, 1H, H-6), 7.23 (d, *J* = 9.0 Hz, 1H, H-3"), 4.17 (s, 2H, 3-N*H*₂) ppm.

In an embodiment, the synthesis of (5-nitro-1,3-phenylene)bis[(2-hydroxyphenyl)methanone] **(4),** (5-nitro-1,3-phenylene)bis[(2-hydroxy-5-nitrophenyl)methanone] **(5),** (5-nitro-1,3-phenylene)bis[(5-chloro-2-hydroxy-4-methylphenyl)methanone] **(6),** comprises the following steps: 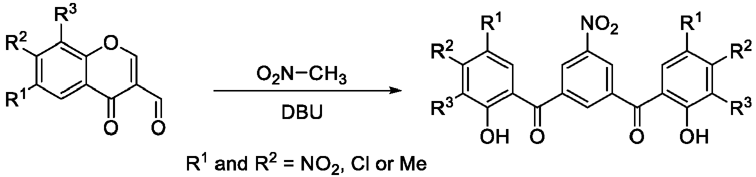 mixing 1 equivalent of 3-formylchromone, 0.5 equivalents of nitromethane and 0.5 equivalents of DBU in dichloromethane; heating the resulting mixture at 40 °C for 20 hours; purifying the obtained crude compounds by silica gel flash column chromatography with an eluent, in particular, hexane/EtOAc (5:1).

The characterization of the compounds (5-nitro-1,3-phenylene)bis[(2-hydroxyphenyl)methanone] **(4),** (5-nitro-1,3-phenylene)bis[(2-hydroxy-5-nitrophenyl)methanone] **(5),** (5-nitro-1,3-phenylene)bis[(5-chloro-2-hydroxy-4-methylphenyl)methanone] **(6)**, is now disclosed.

### (5-Nitro-1,3-phenylene)bis[(2-hydroxyphenyl)methanone] (4)

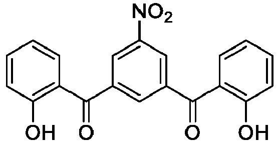

In an embodiment, the characterization of the (5-nitro-1,3-phenylene)bis[(2-hydroxyphenyl)methanone] compound **(4)** is as follows: light yellow solid, 53% yield and a m.p. of 114-116 °C. Further ¹H NMR (300.13 MHz, CDCl₃, 25 °C): δ 11.63 (s, O*H,* 2H), 8.72 (d, *J* 1.5 Hz, H-2, 2H), 8.27 (t, *J* 1.5 Hz, H-4, 1H), 7.60 (ddd, *J* 8.7, 7.2, 1.7 Hz, H-9, 2H), 7.50 (dd, *J* 8.2, 1.7 Hz, H-11, 2H), 7.14 (dd, *J* 8.7, 1.1 Hz, H-8, 2H), 6.96 (ddd, *J* 8.2, 7.2, 1.1 Hz, H-10, 2H) ppm. ¹³C NMR (75 MHz, CDCl₃): δ 197.6 (C-5, 2C), 163.6 (C-7, 2C), 148.0 (C-1), 139.7 (C-3, 2C), 137.8 (C-9, 2C), 134.5 (C-4), 132.7 (C-11, 2C), 126.4 (C-2, 2C), 119.5 (C-10, 2C), 119.1 (C-8, 2C), 118. (C-6, 2C) ppm. HRMS (ESI⁻): *m*/*z* calcd. for [C₂₀H₁₃NO₆-H]⁻ 362.0665; found 362.0662.

### (5-Nitro-1,3-phenylene)bis[(2-hydroxy-5-nitrophenyl)methanone] (5)

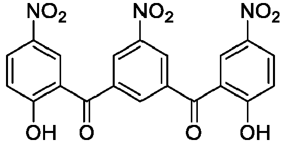

In an embodiment, the characterization of the (5-nitro-1,3-phenylene)bis[(2-hydroxy-5-nitrophenyl)methanone] compound **(5)** was obtained as a beige solid, 86% yield and a m.p. of 256-258 °C. Further ¹H NMR (300.13 MHz, CDCl₃, 25 °C): δ 8.63 (d, *J* 1.6 Hz, H-2, 2H), 8.42 (t, *J* 1.6 Hz, H-4, 1H), 8.36-8.30 (m, H-9 and H-11, 4H), 7.14 (d, *J* 9.8 Hz, H-8, 2H) ppm. ¹³C NMR (75 MHz, DMSO-d₆): δ 192.1 (C-5, 2C), 162.8 (C-7, 2C), 148.5 (C-1), 139.9 (C-1, 2C), 139.0 (C-3, 2C), 134.9 (C-4), 129.4 (C-9 or 11, 2C), 127.9 (C-9 or 11, 2C), 127.4 (C-2, 2C), 125.2 (C-6, 2C), 118.1 (C-8, 2C) ppm. HRMS (ESI⁻): *m*/*z* calcd. for [C₂₀H₁₁N₃O₁₀-H]⁻ 452.0365; found 452.0369.

### (5-Nitro-1,3-phenylene)bis[(5-chloro-2-hydroxy-4-methylphenyl)methanone] (6)

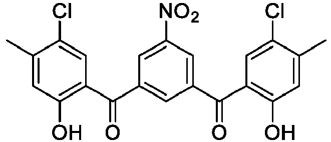

In an embodiment, the characterization of the (5-nitro-1,3-phenylene)bis[(5-chloro-2-hydroxy-4-methylphenyl)methanone] **(6)** was obtained as a yellow solid, 70% yield and a m.p. 159-161 °C. Further ¹H NMR (300.13 MHz, CDCl₃, 25 °C): δ 11.48 (s, 7-O*H,* 2H), 8.69 (d, *J* = 1.5 Hz, H-2, 2H), 8.23 (t, *J* = 1.5 Hz, H-2, 1H), 7.44 (s, H-11, 2H), 7.01 (s, H-8, 2H), 2.42 (s, 9-C*H*₃, 6H) ppm. ¹³C NMR (75 MHz, CDCl₃) δ 196.2 (C-5, 2C), 161.9 (C-7, 2C), 148.0 (C-9, 2C), 147.5 (C-1), 139.5 (C-3, 2C), 134.0 (C-4), 131.7 (C-11, 2C), 126.4 (C-2, 2C), 125.0 (C-10, 2C), 121.1 (C-8, 2C), 117.1 (C-6, 2C), 21.1 (9-*C*H₃, 2C) ppm. HRMS (ESI⁻): *m*/*z* calcd. for [C₂₂H₁₅Cl₂NO₆-H]⁻ 458.0198; found 458.0191.

In an embodiment, compounds 4-6, 10-12 and 14 were prepared as previously described⁵.

### (5-nitro-1,3-phenylene)bis[(2-hydroxy-5-methyl-3-nitrophenyl)methanone] (14)

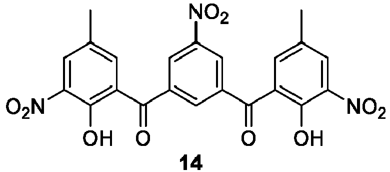

In an embodiment, the characterization of the (5-nitro-1,3-phenylene)bis[(2-hydroxy-5-methyl-3-nitrophenyl)methanone] compound (14) was obtained as dark yellow solid, 77% yield and a m.p. of 256-258 °C. Further ¹H-NMR (300 MHz, CDCl₃); δ = 10.94 (s, 2H, 7-OH), 8.77 (d, *J* = 1.6 Hz, 1H, H-4), 8.60 (s, 1H, H-4), 8.52 (t, *J* = 1.6 Hz, 2H, H-2), 8.20 (d, *J* = 2.4 Hz, 2H, H-9), 7.70 (d, *J* = 2.4 Hz, 2H, H-11), 1.65 (s, 6H, 10-C*H*₃) ppm.

### (5-amino-6-phenylpyridin-3-yl)(2-hydroxy-5-methyl-3-nitrophenyl)methanone (13)

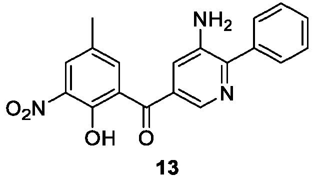

In an embodiment, the characterization of the (5-amino-6-phenylpyridin-3-yl)(2-hydroxy-5-methyl-3-nitrophenyl)methanone compound **(13)** was obtained as dark yellow solid, 85% yield and a m.p. of 240-244 °C. Further ¹H-NMR (300 MHz, DMSO-*d₆*); 10.75 (s, 1H, 2'-OH)8.20 (d, *J* = 1.8 Hz, 1H, H-2 or H-4), 8.03 (d, *J* = 2.25 Hz, 1H, H-6' or H-4'), 7.73 (d, *J* = 1.8 Hz, 1H, H-2 or H-4), 7.71 (t, *J* = 1.5 Hz, 1H, H-4‴), 7.61 (d, *J* = 2.25 Hz, 1H, H-6' or H-4'), 7.54 - 7.42 (m, 4H, H-2‴, H-3‴, H-5‴ and H-6‴), 5,43 (s, 3-N*H*₂), 2.36 (s, 1H, 5'-C*H*₃) ppm.

### In vitro HDAC inhibitory capacity of the compounds

*In vitro* total HDAC activity was measured according to manufacturer's instructions using a nuclear extract from HeLa cells (human cervical cancer cell line) as HDAC source incubated with the Fluor-de-Lys^{®} substrate (50 µM) for 20 min at room temperature (Fluor-de-Lys^{®}, Enzo^{®} Life Sciences, Antwerpen, Belgium). Reaction was stopped by addition of a developing solution (Fluor-de-Lys^{®} developer I concentrate, Enzo^{®} Life Sciences) containing trichostatin A (TSA) (2 µM). After 15 min of incubation at room temperature, fluorescence signal was measured on a fluorescence microplate reader (SYNERGY HTX multi-mode reader) with an exciting wavelength of 360 nm and emission wavelength of 460 nm. All dilutions of tested compounds were prepared in dimethylsulfoxide (DMSO).

*In vitro* HDAC8 isoenzyme activity was tested using 1 U/well of recombinant protein HDAC8 (Enzo^{®} Life Sciences) incubated with the Fluor-de-Lys^{®}-HDAC8 substrate (50 µM) for 20 min at room temperature. Reactions were stopped by adding a developer II solution (Fluor-de-Lys^{®} developer II concentrate, Enzo^{®} Life Sciences) containing 2 µM of TSA. After 45 min of incubation at room temperature, fluorescence is measured as for total HDAC activity.

Representative examples of 3-aminopyridines and 3,5-disubstituted nitrobenzenes were screened for their total HDAC inhibition capacity at 100 µM **(Table 2).** It was observed that only nitro-containing examples were active in the total HDAC screening assay (compounds **1, 3, 9** and **5),** which moved forward to the specific HDAC8 isoenzyme assay **(Table 3).**

The results obtained from the HDAC8 specific assay showed that the IC₅₀ values ranged from 27 to 74 µM, being the most active compound the full nitro-containing one (compound **5**) (IC₅₀ = 27 µM).

**Table 2: In vitro screening of 3-aminopyridines and 3,5-disubstituted nitrobenzenes on total HDAC activity.**

| **Compound** | **%Inhibition at 100 µM** |
|---|---|
| ***3-Aminopyridines*** | |
| 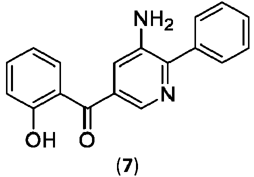 | Inactive |
| 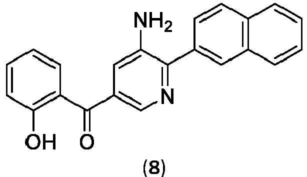 | Inactive |
| 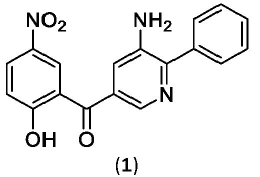 | 78 |
| 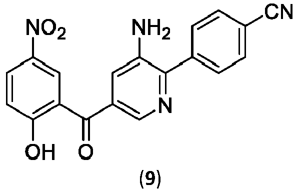 | 82 |
| 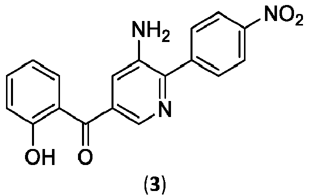 | 67 |
| 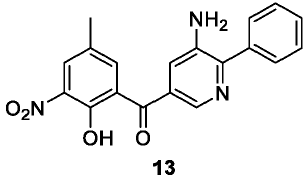 | 67 |

| ***3,5-Disubstituted Nitrobenzenes*** | |
|---|---|
| 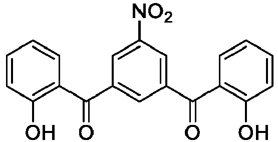 | Inactive |
| 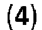 | |
| 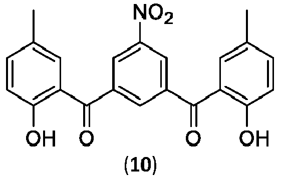 | Inactive |
| 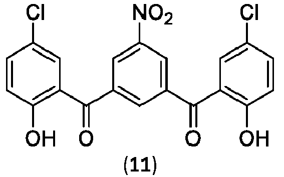 | Inactive |
| 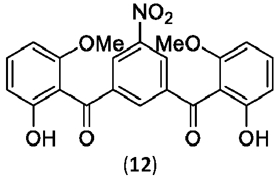 | Inactive |
| 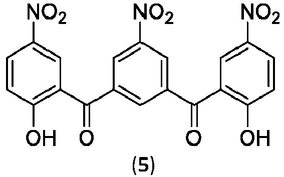 | 73 |
| 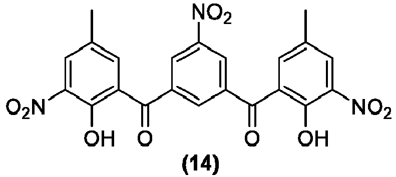 | 65 |
| Trichostatin A | 90^{a} |

| | |
|---|---|
| *^{α} %Inhibition of 1µM* | |

**Table 3: In vitro inhibition of HDAC8 isoenzyme by nitro-containing compounds.**

| **Compound** | **IC₅₀ (µM)** |
|---|---|
| ***3-Aminopyridines*** | |
| 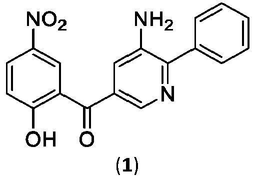 | 74 |
| 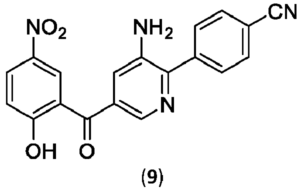 | 58 |

| ***3,5-Disubstituted Nitrobenzenes*** | |
|---|---|
| 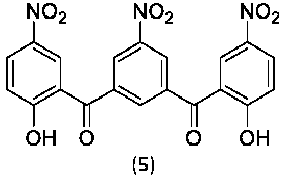 | 27 |

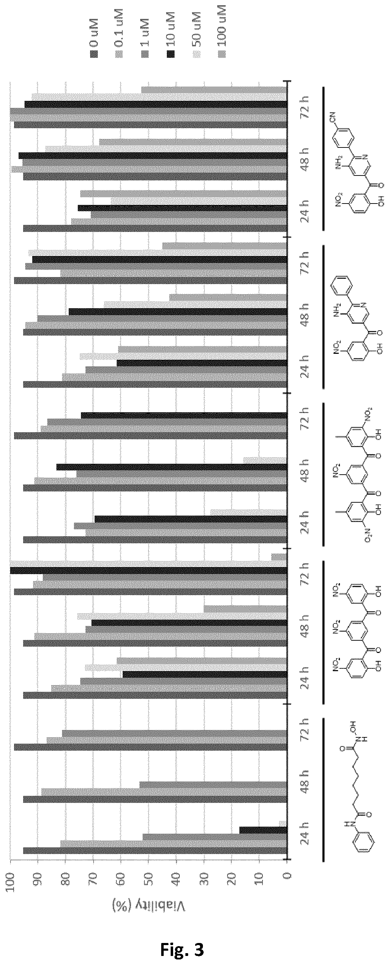

In an embodiment, the effect of the nitrobenzene and 3-aminopyridine derivatives of the present disclosure was evaluated on cell viability, namely on human Jurkat leukaemia cells. Vorinostat was used as positive control. As shown in Figure 3, although both compounds demonstrate anticancer effect, nitrobenzenes decreased the cellular viability more than 3-aminopyridines.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The following dependent claims further set out particular embodiments of the disclosure.

### References

(1) Ho, T. C. S.; Chan, A. H. Y.; Ganesan, A. J. Med. Chem. 2020, 63, 12460.
(2) Seidel, C.; Schnekenburger, M.; Dicato, M.; Diederich, M. Cancer Lett. 2014, 343, 134.
(3) Borretto, E.; Lazzarato, L.; Spallotta, F.; Cencioni, C.; D'Alessandra, Y.; Gaetano, C.; Fruttero, R.; Gasco, A. ACS Med. Chem. Lett. 2013, 4, 994.
(4) Mandegar, M. A.; Patel, S.; Bhatt, U.; Ding, P.; Holan, M.; Lee, J.; Li, Y.; Medina, J.; Nerurkar, A.; Seidl, F.; Sperandio, D.; Widjaja, T.; Wang, X.; "5-Fluoronicotinamide derivatives and uses thereof", 2020; WO2021067859A1.
(5) Francisco, T. N.; Sousa, J. L. C.; Guieu, S.; Silva, A. M. S.; Albuquerque, H. M. T. Synlett 2022, 33, 1505-1510.
(6) Rodrigues, D. A.; Pinheiro, P. d. S. M.; Sagrillo, F. S.; Bolognesi, M. L.; Fraga, C. A. M., Histone deacetylases as targets for the treatment of neurodegenerative disorders: Challenges and future opportunities. Med. Res. Rev. 2020, 40, 2177-2211.

## Claims

1. Compound of formula I or formula II, or a pharmaceutically acceptable salt, or ester or solvate thereof 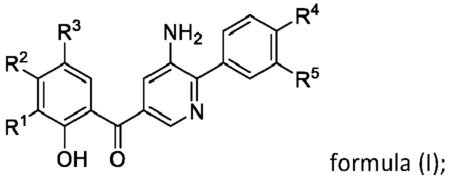 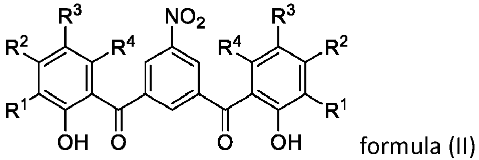 wherein
R¹, R², R³, R⁴ and R⁵ are independently selected from each other;
R¹, R², R³, R⁴ and R⁵ are selected from H, F, Cl, Br, I, CN, NH₂, NO₂, OCH₃, CH₃ or CH₂CH₃;
provided that at least R¹, or R², or R³, or R⁴ or R⁵ is NO₂; preferably at least two of R¹, or R², or R³, or R⁴ are NO₂; more preferably at least three of R¹, or R², or R³, or R⁴ are NO₂;
for use in medicine; preferably R¹ is H.

2. Compound of formula I, or a pharmaceutically acceptable salt, or ester or solvate thereof 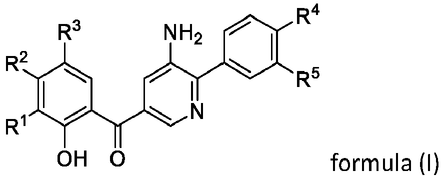 wherein
R¹, R², R³, R⁴ and R⁵ are independently selected from each other;
R¹, R², R³, R⁴ and R⁵ are selected from H, F, Cl, Br, I, CN, NH₂, NO₂, OCH₃, CH₃ or CH₂CH₃; ; preferably R¹ is H;
provided that at least R¹, or R², or R³, or R⁴ or R⁵ is NO₂; preferably at least two of R¹, or R², or R³, or R⁴ are NO₂; more preferably at least three of R¹, or R², or R³, or R⁴ are NO₂.

3. Compound according to any of the previous claims 1-2 wherein R³ is NO₂; and R¹, R², R⁴ and R⁵ are selected from H, F, Cl, Br, I, CN, NH₂, OCH₃, CH₃ or CH₂CH₃.

4. Compound according to any of the previous claims 1-2 wherein R⁴ is NO₂ and R¹, R², R³ and R⁵ are selected from H, F, Cl, Br, I, CN, NH₂, OCH₃, CH₃ or CH₂CH₃.

5. Compound according to any of the previous claims 1-2 wherein and R³ and R⁴ are NO₂.

6. Compound according to any of the previous claims 1-2 wherein the compound is 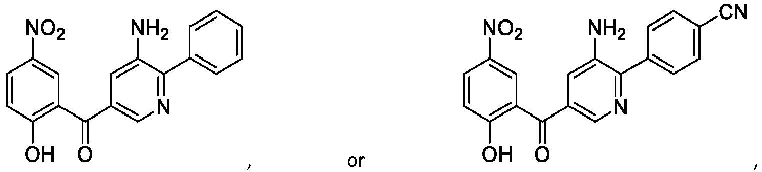 or 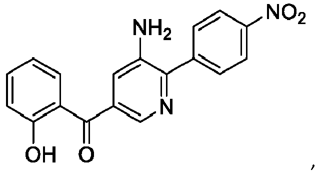 or

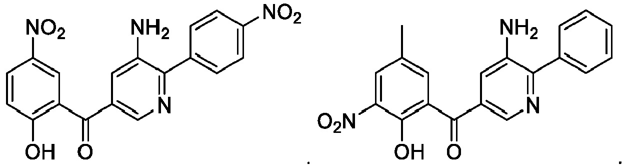

7. Compound for use according to claim 1 wherein R³ is NO₂ and R², R⁴ are selected from H, F, Cl, Br, I, CN, NH₂, OCH₃, CH₃ or CH₂CH₃.

8. Compound for use according to the previous claim wherein R³ is NO₂.

9. Compound for use according to any of the previous claims 7-8 wherein R³ is NO₂ and R⁴ is CH₃.

10. Compound for use according to any of the previous claims 7-9 wherein the compound is

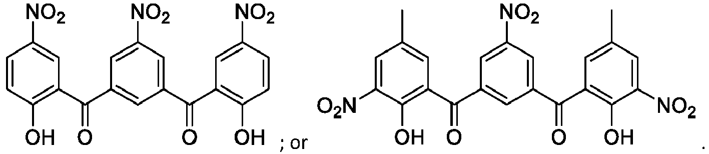

11. Compound according to any of the previous claims 2-6 for use in medicine.

12. Compound according to any of the previous claims 1-11 for use in the treatment or prevention of any condition susceptible of being improved or prevented by selective inhibition of histone deacetylases; preferably by selective inhibition of histone deacetylase 8.

13. Compound according to any of the previous claims 11-12 for use in the prevention or treatment of a neoplasia or for use in the prevention or treatment of a degenerative disease, preferably for use in the prevention or treatment of cancer or a neurodegenerative disease.

14. Pharmaceutical composition comprising the compound as described in any of the previous claims 1-10 and a pharmaceutically acceptable carrier, wherein the compound is in a therapeutically effective amount.

15. Method of obtaining the compound according to any of the previous claims 2- 10 comprising the following steps:
mixing the a 3-formylchromone, the a Kröhnke salt and ammonium acetate in an organic solvent; preferably absolute alcohol; more preferably absolute ethanol;
heating the resulting mixture, preferably under microwave irradiation; wherein the temperature of heating ranges from 90-200 °C, preferably 90 - 180 °C; and in a period ranging from 5-15 min; preferably 110 °C during 10 min, to obtain the crude solution;
evaporating the organic solvent from the crude solution to obtain dried crude; purifying the obtained dried crude to obtain pure compound; preferably purifying by silica gel flash chromatography.
